Europäisches Patentamt

European Patent Office

Office européen des brevets

(11) Publication number: **0 001 179**
A1

# EUROPEAN PATENT APPLICATION

(21) Application number: 78300358.5

(22) Date of filing: 07.09.78

(51) Int. Cl.²: **C 07 B 29/00**
C 07 C 1/00, C 07 C 11/12
C 07 C 11/06, C 07 C 9/14
C 07 C 11/08, C 07 C 120/00
C 07 C 121/16, C 07 C 121/18
C 07 C 37/00, C 07 C 39/12

(30) Priority: 12.09.77 US 832260

(43) Date of publication of application:
21.03.79 Bulletin 79/6

(84) Designated contracting states:
BE CH DE FR GB NL SE

(71) Applicant: THE STANDARD OIL COMPANY
Patent and License Division Midland Building
Cleveland, Ohio 44115(US)

(72) Inventor: Callahan, James Louis
R.D. 6, Township Rd.
101, Wooster Ohio(US)

(74) Representative: Baillie, Iain Cameron et al,
c/o Ladas, Parry, von Gehr. Goldsmith & Deschamps
Blumenstrasse 48
D-8000 München 2(DE)

(54) A process for producing an organic compound by extrusion of sulfur from sulfur containing organic compounds.

(57) Sulfur is "extruded" from various sulfur-containing organic compounds by heating the compounds at a temperature of 200-700°C. in the presence of a sulfur-acceptor.

EP 0 001 179 A1

# A PROCESS FOR PRODUCING AN
# ORGANIC COMPOUND BY
# EXTRUSION OF SULFUR
## FROM SULFUR CONTAINING ORGANIC COMPOUNDS

0001179

U. S. 3,929,859 discloses a process for extruding sulfur from a thiodipropionitrile of the formula NC-R''-S-R'''-CN to produce a dinitrile of the formula NC-R-R'-CN wherein R, R', R'' and R''' are aliphatic or aromatic hydrocarbon radicals; and wherein R and R'' have the same number of carbon atoms and R' and R''' have the same number of carbon atoms. The process described in this patent, however, is limited to producing dinitriles from thiodinitriles.

The present invention provides a process for the extrusion of sulfur from sulfur-containing organic compounds other than thiodinitriles by contacting said sulfur-containing organic compounds at a temperature of 200-700°C. with a sulfur acceptor.

The sulfur-containing organic compound which can be reacted in accordance with the present invention can be described by the following formula

$$R-S-R'$$

wherein R and R' are the same or different unsubstituted or substituted aliphatic or aromatic hydrocarbon radicals. Each of R and R' can be independently substituted with a wide variety of different radicals such as cyano, chlorine, bromine, iodine, fluorine, hydroxy, alkoxy, carboxyl, carboxylate. Also, each of R and R' preferably has 12 or less carbon atoms, more preferably 8 or less carbon atoms. Examples of hydrocarbon substituents found useful as R and/or R' are methyl, ethyl, allyl, butyl, hydroxyphenyl, cyanomethyl, methyl propionyl, cyanoethyl and the like. Preferred R and R' groups are methyl, allyl, butyl, hydroxyphenyl, cyanomethyl, cyanoethyl and 0001179 methyl propionyl.

The present process is carried out by contacting the sulfur-containing organic compound with the sulfur-acceptor at a reaction temperature from about 200-700°C. Within this broad temperature range, temperatures between 300-650°C have been shown to give more desirable results, with temperatures in the range of 400-600°C. being especially useful.

Within the temperature ranges claimed, the reaction of the present invention can be conducted in the vapor or liquid phase. Of special interest in the present invention are reactions conducted in the vapor phase. Of course, to obtain a liquid phase reaction within the higher temperature ranges of the invention, pressure must be applied. It is anticipated that a reactor pressurized with an inert gas, hydrogen and/or acrylonitrile would give the most desirable results.

It will also be appreciated by those skilled in the art that the mode of the reaction (i.e. vapor phase or liquid phase) depends at least to some extent on the sulfur-containing organic compound selected as the starting material. A sulfur-containing organic compound with a relatively great number of carbon atoms cannot be vaporized under practical reaction conditions at temperatures approaching 700°C. Thus, there is a practical limit on the molecular size of sulfur-containing organic compounds which can be reacted in the vapor phase. This practical limit can be readily determined by those skilled in the art by routine experimentation. As a rough guide, it has been found that vapor phase reactions become difficult when the R or R' group in the molecule of the sulfur-containing organic compound has more than about 8 carbon atoms.

The sulfur-acceptor useful in accordance with the present invention can be selected from a wide variety of different materials. Most suitably used are solids which are composed of a metal or metal-containing material.

Among the metals that may be employed in the reaction, those selected from elements of Group IIIA, IVA, VA, VIA, IB, IIB, VB, VIB, VIIB or VIII are preferred. Of these numerous metals, it has been found that iron, nickel, manganese, copper, silver and tin are of greatest interest with the use of iron or copper being of greatest significance because of the especially desirable yields obtained using these solids. These metals may be used alone or in alloys or mixtures.

The reaction conditions other than the temperature limitation are not critical. However, there are certain preferred aspects of the reaction to give most desirable results.

In the vapor phase reactions, it has been found that the use of an additional gas in the reactant feed is desirable to purge the products through the reactor. When a heterogenous solid is employed, this purge gas is also believed to draw the products off the surface of the solid and into the effluent from which the dinitrile is recovered. Essentially any gas may be employed in the reaction. Representative examples of these purge gases include nitrogen, air, acrylonitrile, carbon monoxide, carbon dioxide, argon, hydrogen, methanol and the like. Of special interest as far as the purge gases are concerned is the use of hydrogen, acrylonitrile or mixture thereof. These gases appear to not only act as a purge gas but also act as promoters of the reaction of the sulfur-containing organic compounds to give desirably high yields.

The pressure employed in the reaction may vary widely. Subatmospheric, superatmospheric or atmospheric pressure may be employed. The contact time may range from less than a second to a number of hours depending on the reaction temperature and state of reaction.

The reactor employed in the invention can be an open tube with an inlet for reactants and an outlet for products, the tube being at least partially filled with a sulfur-acceptor. The reactor can take the form of either a fixed-bed reactor with a solid fixed in the reaction zone or a fluid-bed reactor. Although a fixed-bed reactor has been employed in all of the experiments of the Specific Embodiments, it is anticipated that a fluid-bed reactor could be employed to great advantage in the present invention.

When a heterogeneous solid is employed, there is a tendency for the heterogeneous solid to become sulfided during the course of the reaction. This sulfiding is especially noticeable when a metal is employed. As the catalyst becomes sulfided, it tends to lose activity for the desired reaction. Even though some catalysts are enhanced by partial sulfiding, there is a point of sulfiding at which regeneration appears to be desirable. Normally, the regeneration involves at least partial return of the sulfided metal to the metallic state or a lower valence state. This can be suitably accomplished by the action of hydrogen or another reducing agent on the catalyst or in a more convenient method, the catalyst may be oxidized with air and then reduced with a reducing agent. Using either technique, the catalytic activity of the heterogeneous solid has been observed to be restored.

The recovery and purification of the extrusion product is normally very convenient. The reactor effluent contains unreacted sulfur-containing organic compound, the extrusion product and various organic compounds derived by breaking the sulfur out of the sulfur-containing organic compound without joining the carbon atom of the R and R' groups. These different materials can be separated from one another by conventional techniques, such as distillation and the like.

The present invention finds significant use in the manufacture of various different compounds. For example, it is possible in accordance with the present invention by suitably selecting the starting material to produce n-butane, 1,5-hexadiene, n-octane, 4,4'-dihydroxy-diphenol, propionitrile, dimethyl adipate and the like. As is well known, these different compounds have various different uses, such as solvents, dielectrics, ingredients for polymers.

Example 1.

A reactor was constructed of an 8.0 mm inside diameter stainless steel tube. The reactor had a 10 cc reaction zone, an inlet for reactants and an outlet for products. The reactor was filled with a catalyst acceptor comprising 3 cc of fine copper lathe turnings.

$H_2$ was fed to the reactor at a rate of 15 STP cc/min. for the entire period of the test. 1.0 cc Diallyl sulfide was fed to the reactor with the hydrogen uniformly over a period of 5 minutes. The reaction temperature was 537°C. The reaction product was recovered, and it was found that the total conversion of diallyl sulfide was 62.4% with the selectivity to the expected extrusion product, hexadiene-1,5, being 0.85% and the selectivity to propylene and a large number of unidentified hydrocarbons being about 99%.

For the purposes of this application, the following definitions are used:

$$\% \text{ Conversion} = \frac{\text{moles of feed reacted}}{\text{moles of feed fed}} \times 100$$

$$\% \text{ Selectivity} = \frac{\text{moles of product formed}}{\text{moles of feed reacted}} \times 100$$

Example 2 - Dibutylsulfide.

Example 1 was repeated except that the feed consisted of dibutylsulfide rather than diallylsulfide. The conversion of dibutylsulfide was found to be 60.3% the selectivity to the desired extrusion product n-octane was 0.6% and the selectivity to butylenes and a large number of unidentified hydrocarbons was about 99%.

Example 3 - 4,4-Thiodiphenol.

Example 1 was repeated except that the feed consisted of 0.4 grams 4,4-thiodiphenol diluted with 2 cc acrylonitrile, the reaction temperature was 524°C, the feed was fed to the reactor over a period of 10 minutes, and the acceptor comprised 2 cc 9-40 mesh particles of sintered copper powder. The conversion of 4,4-thiodiphenol was found to be 100%, the selectivity to the expected extrusion product 4,4'-dihydroxy-diphenol was found to be 3.3% and the selectivity to phenol was found to be greater than 50%.

Example 4 - Methylthioacetonitrile.

Example 3 was repeated except that the feed comprised 1.0 cc methylthioacetonitrile and the sulfur acceptor comprised 4 cc of 9-40 mesh particles of sintered copper powder. The total conversion of methylthioacetonitrile was found to be about 30%, the selectivity to the expected extrusion product, propionitrile, was found to be 1.3% and the selectivity to acetonitrile was found to be 68.3%.

Example 5 - Dimethylthiodipropionate.

Example 3 was repeated except that the feed comprised 0.2 grams dimethylthiodipropionate diluted with 2 cc methanol, and the sulfur acceptor comprised 3 cc fine copper lathed turnings. The total conversion of dimethylthiodipropionate was found to be 10.3%, the selectivity to the desired extrusion product, dimethyladipate, was found to be 1.0%, the selectivity to methylacrylate was 48.8%, the selectivity to propionic acid was 14.6% and the selectivity to acrylic acid was 36.6%.

Example 6 - Dimethylthiodipropionate.

Example 5 was repeated except that the acceptor comprised 3 cc of 1/8" diameter porous iron chips. The total conversion of dimethylthiodipropionate was found to be 11.8%, the selectivity to the desired extrusion product dimethyladipate was found to be 1.1, the selectivity to methylacrylate was 47.1%, the selectivity to propionic acid was 20.0%, and the selectivity to acrylic acid was 31.8%.

Although only a few embodiments of the present invention have been shown, it should be appreciated that many modifications can be made without departing from the spirit and scope of the invention. All such modifications are intended to be included within the scope of the present invention, which is to be limited only by the following claims.

CLAIMS

1.      A process for producing an organic compound, other than a dinitrile, of the Formula R-R' from a sulfur-containing organic compound of the formula R-S-R' wherein R and R' are the same or different unsubstituted or substituted aliphatic or aromatic hydrocarbon radicals, characterized by contacting said sulfur-containing organic compound with a sulfur acceptor at a temperature of from 200-700°C.

2.      A process according to claim 1, characterized in that the temperature of reaction is 300-650°C.

3.      A process according to claim 1, characterized in that the temperature of reaction is 400-600°C.

4.      A process according to any one of claims 1 to 3, characterized in that said process is conducted in the vapor phase.

5.      A process according to any one of the preceding claims, characterized in that, each of R and R' are independently unsubstituted or substituted with the same or different substituent selected from the group consisting of cyano, chlorine, bromine, iodine, fluorine, hydroxy, alkoxy, carboxyl and carboxylate.

6.      A process according to claim 5, characterized in that each of R and R' has not more than 12 carbon atoms.

7.      A process according to any one of the preceding claims, characterized in that said sulfur acceptor is a metal or mixtures thereof.

8.      A process according to claim 7, characterized in that said metal or mixtures thereof are selected from Groups IIIA, IVA, VA, VIA, IB, IIB, VB, VIB, VIIB or VIII of the Periodic Table.

9.      A process according to claim 7, characterized in that said metal or mixtures thereof is selected from iron, nickel, manganese, copper silver and tin.

0001179

10.     A process according to claim 9, characterized in that said metal is iron or copper.

0001179

# EUROPEAN SEARCH REPORT

| | DOCUMENTS CONSIDERED TO BE RELEVANT | | CLASSIFICATION OF THE APPLICATION (Int. Cl.²) |
|---|---|---|---|
| **Category** | Citation of document with indication, where appropriate, of relevant passages | Relevant to claim | |
| | US 3 424 784 (G. BARSKY) <br> * Whole document * <br><br> -- | 1-10 | C 07 B 29/00 <br> C 07 C 1/00 <br> 11/12 <br> 11/06 <br> 9/14 <br> 11/08 <br> 120/00 <br> 121/16 <br> 121/18 <br> 37/00 <br> 39/12 <br> 39/04 <br> 67/30 ./. |
| D | US 3 929 859 (J.L. CALLAMAN) <br> * Claims 1-15 * <br><br> -- | 1-10 | |
| A | US 3 185 743 (E.M. LA COMBE et al.) <br> * Column 1, line 27 - column 2, line 57 * <br><br> -- | 1 | TECHNICAL FIELDS SEARCHED (Int.Cl.²) |
| A | W. TEILHEIMER "Synthetic Methods of Organic Chemistry", vol. 25, page 502, paragraph 691 (1971) S. Karger, Basel etc. <br><br> ---- | 1 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 15-12-1978 | SAGATYS |

European Patent Office

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | | | C 07 C 69/44 69/54 51/00 53/08 53/22 |

TECHNICAL FIELDS SEARCHED (Int. Cl.³)